# EUROPEAN PATENT SPECIFICATION

(11) **EP 2 967 331 B1**
(45) Date of publication and mention of the grant of the patent: **25.12.2019**
(21) Application number: 14710540.7
(22) Date of filing: 13.03.2014
(51) Int. Cl.: A61B 5/00

(54) **MEASURING WHEALS AND DETECTING ALLERGIES**
MESSUNG VON URTICA UND NACHWEIS VON ALLERGIEN
MESURE DE PAPULES ET DÉTECTION D'ALLERGIES

(30) Priority: 13.03.2013 ES 201330353
(43) Date of publication of application: 20.01.2016
(73) Proprietor: INNOPRICK, S.L., 20009 San Sebastián (Gipuzkoa) (ES)
(72) Inventor: GÓMEZ MARTÍN, Eduardo, E-20018 San Sebastián (ES); GASTAMINZA LASARTE, Gabriel, E-31009 Pamplona (ES); JUSTO, Xabier, E-20018 San Sebastián (ES); GIL, Jorge Juan, E-20018 San Sebastián (ES); DÍAZ, Iñaki, E-20018 San Sebastián (ES)
(74) Representative: ZBM Patents - Zea, Barlocci & Markvardsen
(86) International application number: PCT/EP2014/055019
(87) International publication number: WO 2014/140215

(56) References cited:
- CA-A1- 2 828 785
- US-A1- 2012 253 224
- R. V. DOS SANTOS ET AL: "Beyond flat weals: validation of a three-dimensional imaging technology that will improve skin allergy research", CLINICAL AND EXPERIMENTAL DERMATOLOGY, vol. 33, no. 6, 1 November 2008 (2008-11-01), pages 772-775, XP055030617, ISSN: 0307-6938, DOI: 10.1111/j.1365-2230.2008.02897.x
- ROQUES C ET AL: "PRIMOS: an optical system that produces three-dimensional measurements of skin surfaces", JOURNAL OF WOUND CARE, MACMILLAN, LONDON, GB, vol. 12, no. 9, 1 October 2003 (2003-10-01), pages 362-364, XP008161027, ISSN: 0969-0700
- HEIKKI LAMMINEN ET AL: "Computer-aided skin prick test", EXPERIMENTAL DERMATOLOGY, vol. 17, no. 11, 1 November 2008 (2008-11-01), pages 975-976, XP055120513, ISSN: 0906-6705, DOI: 10.1111/j.1600-0625.2008.00749.x

## Description

The present disclosure relates to allergy detection and more specifically to methods and devices for measuring wheals and devices for detecting allergies.

### BACKGROUND

An allergy is a reaction of the human body when it comes in contact with a substance called allergen. This may provoke the appearance of a number of symptoms, such as rhinitis, itching, asthma, urticaria, etc., of varying severity, ranging from mild discomfort to severe health conditions that may even endanger a person's life.

Any sort of substance may cause an allergic reaction in people, by coming into contact with the patient in a variety of ways. For example, it may be a suspended substance found in the environment and breathed in by a person or patient, such as pollen or plant animal secretions, or through ingestion, as in the case of food or medicines.

The main remedy for an allergic person is to avoid contact with the allergen, especially in case of allergies which can be particularly severe. However, it is not always possible to completely isolate a patient from the allergen. Various treatments for allergies exist, following which the patient may stop showing symptoms to the allergen, thus allowing a normal life.

In order to be able to avoid contact with the allergen and in order to establish an effective treatment, an allergen must be clearly identified first. This is not always straightforward. To this cause, different methods have been developed for detection of allergies.

Typical methods for the detection of allergies involve subjecting the patient to a potential allergen under controlled conditions. Other known methods involve a blood analysis to validate or rule out an allergy.

The most commonly used method for detecting allergies is known as a "prick test". It consists of applying a small amount of allergen to the skin of a patient, usually on the inner forearm or on the back of the patient, then making a micro-incision on the skin with a small knife, thereby bringing the allergen in contact with the body. As a consequence, after a few minutes a small allergic reaction may take place that may form a wheal or papule. Its size and shape determine the degree of affliction.

In a next step, a doctor or nurse marks the contour of the wheal with a pen or marker. This mark is then collected by providing a piece of adhesive tape on the mark so that the ink adheres to the tape. Then the piece of tape is placed on a graph paper, usually in an area of the paper that identifies the corresponding allergen. Then the number of squares of the maximum diameter of the wheal is determined and written down. These results are then transferred to doctor, who determines a suitable prescription and adds it to the patient's record.

This method is often applied simultaneously with different allergens, such that it is possible to simultaneously test multiple allergies.

The "prick test" method is relatively simple and fast, allowing the performance of various tests simultaneously. However, the analysis and measurement of the size of the wheals that correspond to a positive reaction are performed in a totally manual and laborious manner. Furthermore, for the prescription from the part of the doctor it is necessary to send the result on paper to the doctor's office, thus further lengthening and possibly delaying the process. It is also necessary to send the case history in order to properly review the result. Document CA2828785 discloses a device and method for determining a skin inflammation value such as in the case of wheals.

### SUMMARY

In a first aspect, a method of measuring one or more dimensions of one or more wheals on a three-dimensional representation of an area of the skin of a forearm of a person is disclosed. A filtering stage is applied to the three-dimensional representation of the area of the skin of the person to generate a first and a second intermediate representation of the area. Said filtering stage is configured to filter out any irregularities of the three-dimensional representation below a first predetermined size to generate the first intermediate representation. Furthermore, said filtering stage is configured to filter out any irregularities of the three-dimensional representation below a second predetermined size to generate the second intermediate representation. The second intermediate representation of the area is then subtracted from the first intermediate representation of the area to generate a three-dimensional representation of said one or more wheals. Finally, the dimensions of said three-dimensional representation of said one or more wheals are determined.

In this aspect, an automated method for determining dimensions of wheals is provided, which is much quicker and more reliable than prior art methods.

The three-dimensional representation may be a digital representation of the area of the skin of the person. The filtering stage may comprise one or more filters, such as smoothening filters.

In some embodiments, said applying a filtering stage may comprise applying at least a first filtering stage to the three-dimensional representation of the area of the skin of the person to generate the first intermediate representation of the area and applying at least a second filtering stage to the first intermediate representation of the area of the skin to generate the second intermediate representation of the area. In alternative example, the second filtering stage may be applied to the three-dimensional representation instead of the first intermediate representation.

Said first filtering stage may have a first sensitivity that allows the elimination of noise, hair, such as vellus hair, and any other small skin imperfections from the three-dimensional representation. For example, the first predetermined size may be substantially smaller than a minimum size wheal. For example, a first filtering stage may filter out irregularities/imperfections having a length or surface below approximately 3 millimeters or 7 sq. millimeters, respectively. This size may be perceived by medical practitioners as a minimum size for considering a wheal as an indication of an allergic reaction.

The second filter stage may also be a smoothening filter, however stronger, having a second sensitivity that allows the elimination of the wheals from the three-dimensional representation or from the first intermediate representation. The second predetermined size (i.e. a limit dimension of an irregularity or imperfection) may be set to a size larger than the first predetermined size and, preferably, larger than a size that would correspond to a large wheal or a maximum anticipated size of a wheal. This may vary according to the allergy or amount of allergen used.

After applying the filtering stages, a difference between the two intermediate representations may then be the volume of the wheals. By subtracting the second from the first intermediate representation it is thereby possible to generate a three-dimensional representation of only the one or more wheals present in the original three-dimensional representation of the area of the forearm.

Then one or more dimensions may be relatively easily determined or measured. Measuring the size of wheals may then be performed either manually or automatically based on their three-dimensional representation.

In some embodiments, the method may further comprise scanning of the area of the skin with a light beam to generate said three-dimensional representation of the area of the skin. Said scanning may be performed by the same apparatus as that generating the three-dimensional representation of the wheals or by a separate apparatus. The generation of the three-dimensional representation of the area of the skin requires the presence of the forearm of the person. However, the generation of the three-dimensional representation of the wheals and the ensuing measuring of their size may be performed simultaneously or at a later time by the same or another apparatus not requiring the presence of the person.

Some prior art systems are based on using a scan or photo of the forearm before applying the allergen(s) and one after applying the allergen(s). On the contrary, with a method according to these embodiments, only a single scan or a scan in only one direction is required.

In some embodiments, the scanning of the area of the skin with a light beam to generate said three-dimensional representation of the area of the skin may comprise displacing a light beam generator in a direction along the axis of the forearm, emitting said light beam, and applying a three-dimensional triangulation algorithm to generate said three-dimensional representation of the area of the skin. As the light beam generator is displaced along the axis of the forearm, the light beam may sweep the area where the one or more wheals are present. The reflection of said beam may be recorded by a receiving sensor generating a record of the elevation of the skin at distinct points by applying a suitable triangulation algorithm. A grid or cloud of points representing a three-dimensional representation of a portion of the skin may be obtained.

The light beam generator may comprise a light source, such as e.g. a LED or a laser and a suitable lens system.

In some embodiments, the light beam projected onto the skin may correspond to a line projected onto the skin, e.g. a line transverse to a longitudinal axis of a forearm. By emitting a line on the forearm and receiving its reflection at a sensor, it is possible to obtain a map of the distances between the forearm and the beam line generator. A plurality of sections of the forearm may thus be obtained. This represents an efficient manner of obtaining a cloud of points for a portion of the forearm.

In some embodiments, displacing may comprise a controlled speed displacement. This allows associating each light beam with the position on the skin where it is reflected. In other embodiments said displacing may comprises an arbitrary speed displacement. It is then beneficial to register the position of the light beam (e.g. using a suitable encoder) for each section of the forearm in order to associate each light beam with the position on the skin.

In another aspect, a device for measuring wheals is disclosed. The device comprises a resting base configured to receive a forearm of a person in a first direction. The device furthermore comprises a light beam generator for emitting a light beam, the light beam generator being movable along the first directions. The device may furthermore comprise a sensor that is operatively coupled to the light beam generator such as to move with the light beam generator and receive a reflection from the light beam emitted on the forearm. The device is configured to generate a three-dimensional representation of at least a portion of said forearm. The resting base may be ergonomically adjusted to allow the person to rest their arm on the base for an amount of time that may be required for the two-dimensional sensor to generate the three-dimensional representation of the forearm.

In some embodiments, the light beam emitted onto the forearm may correspond to a line projected onto the skin such that a two-dimensional representation of a plurality of sections of the forearm is obtained. The plurality of said two-dimensional representations of sections of said at least one portion of the forearm may form the basis to generate the three-dimensional representation of the area with the use of a triangulation algorithm.

In some embodiments the device may further comprise at least one guide, substantially parallel to the first direction. The light beam generator may be movable along said at least one guide. The guide allows the direction that the light beam generator and corresponding sensor move and scan the forearm to remain substantially constant so that the two-dimensional sections generated as it scans the forearm do not intersect. For example, the guide may be in the form of a rail and the light beam generator may comprise wheels adapted to be coupled to the rail. One skilled in the art may appreciate that any type of guiding system suitable for guiding the light beam generator along the first direction may be used. Furthermore, in one implementation, a pair of parallel guides may be used. In this implementation the two guides may be placed diametrically opposed to the imaginary axis of the forearm and substantially parallel to the first direction. The light beam generator may then be more stable as it passes over the forearm during the scanning process.

In some embodiments, the device may further comprise a frame. The frame may carry a light beam generator and be movable along said at least one guide. In one implementation, the frame may comprise two legs, each leg having one end guidable in a guide, respectively. Each guide may be placed diametrically opposed to the forearm. The other end of the legs may be attached to a head part. The head part may comprise the light beam generator and/or sensor.

In some embodiments, the device may further comprise a motor to displace the light beam generator along said first direction. The motor may cause the light beam generator to hover in the first direction in a steady speed, thus allowing an even scanning of the forearm where each two-dimensional section will be substantially at equal distance with any previous or subsequent section. In some implementations the motor may have each position of the light beam generator and/or sensor registered with a reference system. Therefore, even if no steady speed is accomplished, it may still be possible to register two-dimensional sections.

In some embodiments, the light beam generator may be a laser beam generator and in other embodiments a LED beam generator. Using such light beam generators has the benefit that with one pass or scan and by receiving the reflection with a reflection sensor that may be incorporated in the laser beam line generator it may be possible to obtain a map of distances between the patient skin surface and the laser beam line generator. Knowing said distances it is possible to obtain a three-dimensional representation of the portion of the forearm.

In some embodiments, the device may further comprise an incision tool. The incision tool may be configured to automatically perform an incision at a selected point of the forearm. This allows for controlling the incision size and position so that the results of the measurement process may be comparable and reproducible for the same patient or between patients.

In some embodiments, the incision tool may be movable in a second direction, transversal to the first direction. For example, the incision tool may be attached to the same head unit that bears the two-dimensional sensor and move from the head unit towards the forearm in a direction substantially transversal to the first direction.

In some embodiments, the device may further comprise an allergen dispensing module. The allergen dispensing module may be configured to automatically provide an allergen on the selected point of the forearm. Therefore, the quantity and position of the application of the allergen may be controllable and thus the measurement results may be reproducible for the same patient or even between patients. The allergen dispensing tool may also be movable in a third direction, transversal to the first direction. In some implementations the allergen dispensing tool may be incorporated in the head unit with the two-dimensional sensor and/or with the incision tool.

In some embodiments, the device may further comprise an allergen storage module. In that way the whole allergy test process may be integrated with the same device which may be portable and controllable by a single person.

In some embodiments, the allergen storage module may be thermally controlled to allow preservation of the allergen for a period of time. For example, the allergen storage module may be refrigerated.

In some embodiments the allergen storage module may comprise a plurality of allergen compartments. Each compartment may be coupled to the allergen dispensing tool via a conduit. Therefore, a plurality of allergy tests may be performed by the same device and the patient needs not remove the forearm between tests.

In some embodiments, the allergen storage module may comprise a plurality of replaceable and/or refillable single dose capsules. This may allow, for example, different tests to be performed for different patients or during different seasons.

In some embodiments, the device may further comprise retaining means connected to the resting base. The retaining means may be configured to maintain the forearm in a substantially stationary position on the resting base during said generation of the three-dimensional representation. Although the exact position of the forearm is not critical for the measurement process, it is important to maintain the arm in substantially stationary position so that the three-dimensional representation is as realistic as possible.

In some embodiments, the retaining means may comprise a handle configured to be held by the person's hand. Therefore the patient may control the movement of the arm and not feel restrained.

In some embodiments, the device may further comprise a filtering module. The filtering module may be configured to receive the three-dimensional representation of the portion of the forearm and generate a three-dimensional representation of said one or more wheals. The filtering module may be internal or external to the device. In one implementation, the device may comprise a scanning portion for generating the three-dimensional representation of the forearm and a filtering portion for generating the three-dimensional representation of the wheals. In other implementations the scanning portion may communicate with the filtering portion. The filtering portion may be at a location remote with respect to the scanning portion. The filtering module may form part of the filtering portion.

In some embodiments, the filtering module may be configured to apply a filtering stage to the three-dimensional representation of the portion of the forearm in order to generate a first intermediate representation of the area. Said first intermediate representation (which may be obtained after a first filtering stage) may have filtered out any features of the skin having a size substantially smaller than that of said one or more wheals. The filtering module may also be configured to generate a second intermediate representation of the area. Said second intermediate representation (which may be obtained after a second filtering stage) may have filtered out any features of the skin having a size up to substantially the size of said one or more wheals, e.g. a maximum size, or a standard maximum size. The subtracting module may be configured to subtract the second intermediate representation of the area from the first intermediate representation of the area to generate a three-dimensional representation of said one or more wheals. The filtering module may be a smoothening filter module configured to apply smoothening filters of varying intensity. Therefore, the filtering module may first remove features present in the three-dimensional representation that may be attributable to hair, noise and other skin imperfections and generate the first intermediate representation and subsequently remove even larger features, such as the wheals and generate the second intermediate representation. Therefore, by subtracting the second representation from the first, what remains can only be attributable to the wheals. Thus it may be possible to directly measure the size of the wheals from the three-dimensional representation of the wheals.

In some embodiments, the device may further comprise a computing module, configured to associate the three-dimensional representation of the one or more wheals with one or more applied allergens and generate a measurement report of said three-dimensional representation. The report may include the size of the wheals or may include information that may allow a practitioner to derive the size of the wheal, such as three dimensional views or representations of the wheals in a pre-sized matrix. The measurement report may, for example, comprise an indication of at least the height or the volume of said one or more wheals. The size of the wheal may be an indication of the reaction level of the patient to the allergen.

In another aspect, a computing device is disclosed. The computing device may comprise a memory, e.g. a non-transitory memory, and a processor. The memory may store computer program instructions executable by the processor. Said instructions may comprise functionality to execute a method of measuring one or more wheals on a three-dimensional representation of an area of the skin of a forearm of a person according to embodiments herein.

In yet another aspect, a computer program product is disclosed. The computer program product may comprise instructions that when executed on a computing device causes a computing device to perform any of the examples of the methods of measuring one or more wheals on a three-dimensional representation of an area of the skin of a forearm of a person disclosed herein. The computer program product may be stored in recording media or carried by a carrier signal.

### BRIEF DESCRIPTION OF THE DRAWINGS

Non-limiting examples of the present disclosure will be described in the following, with reference to the appended drawings, in which:
Figure 1 illustrates a flow diagram of an example of a method of measuring one or more wheals.
Figures 2A, 2B and 2C illustrate front, side and top views, respectively, of an example of a device for measuring wheals.
Figure 2D illustrates a side view of the same device for measuring wheals, in use.
Figure 3 illustrates an example of a three-dimensional representation of an area of a forearm after a filtering stage has been applied.
Figures 4A and 4B illustrate a usage scenario of an example of a device for measuring wheals.
Figure 5 illustrates a cross section of the device of Fig. 4A and 4B.
Figure 6 illustrates a cross section of an alternative implementation of the device of Fig. 5.
Figures 7A to 7E illustrate an example of an operating sequence of the device of Fig. 5.

### DETAILED DESCRIPTION

The present disclosure provides examples of methods and devices for measuring wheals and for detecting allergies. They allow analysis and processing of the results obtained by an automated or semi-automated "prick test" for allergies. The proposed methods are partly based on the conventional method for detecting allergies known as a "prick test". However, instead of manually registering on paper an edge of a wheal, wheals may be digitized and integrated into a software application that in some examples may record, analyze and electronically archive the results.

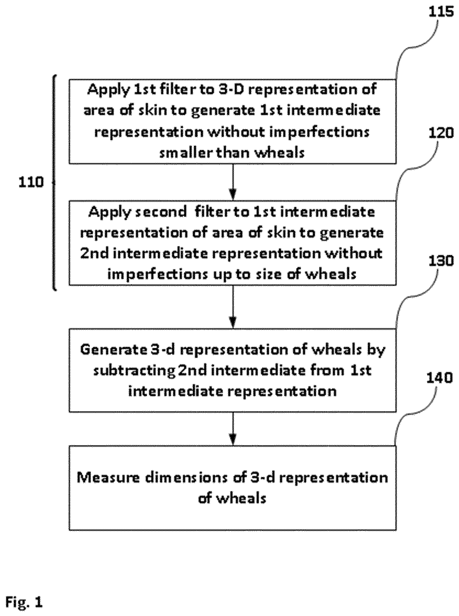

Fig. 1 illustrates a flow diagram of a method of measuring one or more wheals on a three-dimensional representation of an area of the skin of a person, according to an example. During a filtering stage 110, a first filter (or filtering stage) is applied to the three-dimensional representation of the area of the skin of the person in step 115. The purpose of the first filtering stage is to smoothen the three-dimensional representation in such a way as to remove any features that may be attributable to noise, hair or other minor skin imperfections.

Thereby, a first intermediate representation may be generated that may include only larger features that may represent only the one or more wheals of the forearm. Therefore, the first filtering stage may be configured to filter only those features of the skin that have a size substantially smaller than that of said one or more wheals.

A smoothening filter or more than one smoothening filter, filtering out irregularities or imperfections smaller than a predetermined size may be applied. The predetermined size may be e.g. a length of 2 millimeters, small enough to filter out e.g. hairs. In this sense, the application of the first filtering stage (which may comprise a plurality of filtering steps) may include a combination of 2D filters, such as "mean filters" and "median filters".

At block 120, a second filtering stage may be applied to the first intermediate representation of the area of the skin to generate a second intermediate representation of the area. Alternatively, the second filtering stage may be applied to the three-dimensional representation of the area of the skin. The second filtering stage may also be a smoothening filter and may also comprise a plurality of filtering steps. For example, a combination of "mean filters" and "median filters" may be applied.

The second filtering stage may be designed to filter our irregularities/imperfections smaller than a second predetermined size. The second predetermined size may be selected based on the expected size of a wheal. In particular, the second predetermined size may be larger than an expected largest size for a wheal. For example, when an allergen is known to generate wheals of a dimension (length, width or diameter) of 3 mm - 15 mm, the second predetermined size may be selected above the aforementioned higher limit of 15 mm, e.g. may be set at 20 mm, so that all potential wheals may be filtered out by the second filter. One skilled in the art may appreciate that the selection of the second predetermined size may vary according to the allergy, the allergen amount deposited on the skin or other characteristics of the allergy test.

The second filtering stage may thus be different from the first filtering stage so that it may filter out not only the minor features that were or can be filtered out by the first filter but also larger features, such as the wheals, that are presumably left untouched in the first intermediate representation.

A second intermediate representation may thus have filtered out all features of the skin that may have a size up to substantially the (maximum) size of said one or more wheals. If the two filters are properly configured or selected, then the only difference between the two intermediate representations shall be the wheals.

Therefore, at block 130, the second intermediate representation of the area may be subtracted from the first intermediate representation of the area to generate a three-dimensional representation of said one or more wheals. Cancelling out the two intermediate representations allows for the three-dimensional representation of the one or more wheals which enables block 140, which is measuring or otherwise determining the dimensions of the wheals.

A measurement may be performed on the three-dimensional representation of the wheals. The dimensions of the wheals measured may be in one dimension the width or the height, in two dimensions the area or the perimeter and in three dimensions the volume.

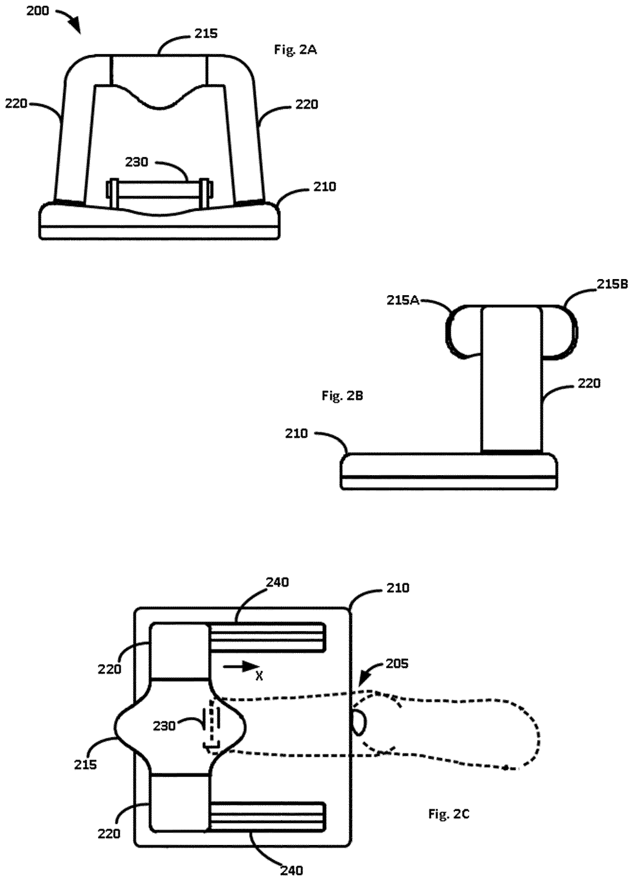

Figures 2A, 2B and 2C illustrate front, side and top views, respectively, of a device for measuring wheals, according to an example. The device 200 may comprise a resting base 210 for receiving the forearm 205 of a patient and for supporting a movable unit of the device.

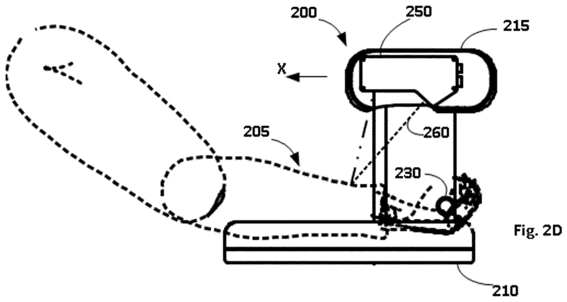

The movable unit may comprises a head unit 215 supported by a pair of columns 220 on the resting base 210. The resting base 210 may comprise a pair of guides 240. Each guide 240 may be configured to receive one of the columns 220 in such a way that the columns and the head unit may be movable in a first direction X as indicated by the arrow in Fig. 2C or 2D.

The head unit 215 may comprises a sensor having a light beam generator 215A and a reflection receiver, i.e. a sensor 215B. As the movable unit moves along the path of the guides, the two-dimensional sensor may scan the forearm and generate a three-dimensional representation of the area of the forearm that it may scan.

Such a three-dimensional representation may be formed by a cloud of points, wherein these points are obtained by a triangulation performed on the received reflection. The point of emission of the light beam is known and the point of reflection at the two dimensional sensor is also known. From this, through suitable methods of triangulation, the point of reflection on the forearm can be determined.

In some examples, the light beam emitted onto the forearm may be a line. Such a line may be formed with a suitable lens system. The source of the light beam may e.g. be a laser or a LED. The three-dimensional representation may thus be obtained by combining a plurality of sections of the forearm obtained by the line projection.

The device 200 may further comprise a handle 230. The patient may hold the handle during the scanning process so that the forearm remains substantially motionless. This allows for minimizing the generation of any errors that may be caused by the movement of the patient.

The handle may be adjustable to account for patients of different forearm size. Thus, the handle may move longitudinally, and may e.g. lock in a suitable position for the forearm of the patient concerned. Such a handle may be manually put in a suitable position by the user and be locked in a predefined interlocking position, or it may be driven by a servomotor or the like to allow locking at any longitudinal position.

Fig. 2D shows the device 200 during operation. A forearm 205 of a person is introduced in the resting base. The person holds the handle 230 in such a way that the anterior side of the forearm faces the head unit 215. When the forearm is substantially motionless, a user of the device, such as hospital personnel, a medical practitioner or a nurse, may turn on the device so that the scanning operation may begin.

The movable unit may then start moving towards the patient. At the same time the two-dimensional sensor 250 emits a line beam 260 towards the forearm 205 of the patient. A reflection receiver 215B of the two-dimensional sensor 250 may then capture the reflection of the beam. As the movable unit moves, a plurality of line beams are captured by the reflection receiver, thus generating a plurality of two-dimensional sections of the area of the forearm being scanned. At the end of the process, the plurality of two-dimensional sections may be used to generate a three-dimensional representation of the area of the forearm 205 of the person.

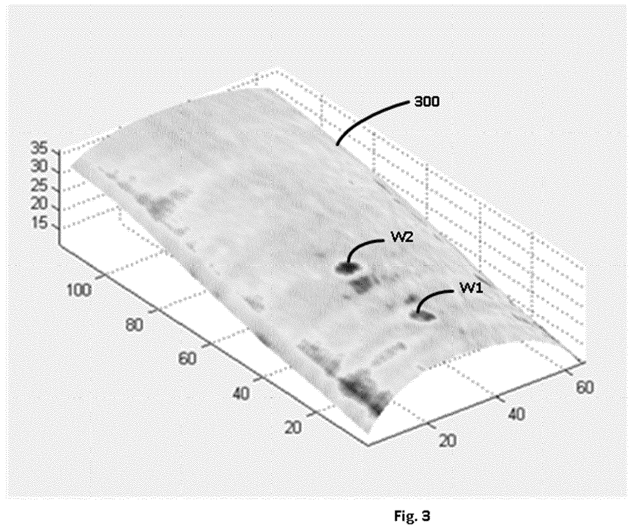

Figure 3 illustrates a three-dimensional representation 300 of an area of a forearm after a filtering stage has been applied. The three-dimensional representation 300 is, therefore, a three-dimensional representation of the wheals, in this particular case visually represented on the forearm. Any minor features in the original three-dimensional representation, such as noise(in the signal), hair or small skin imperfections, have been filtered out by the application of a first smoothening filter or first set of smoothening filters.

Then a second filtering stage applied has removed the wheals and has generated an even smoother second intermediate three-dimensional representation without the wheals being identifiable. Subsequently, a subtraction of the second intermediate three-dimensional representation from the first intermediate three-dimensional representation has left only the three-dimensional representation of the two wheals.

To generate figure 3, the resulting representation of the wheals have been depicted on the first intermediate representation. This merely represents one way of visualizing the wheals.

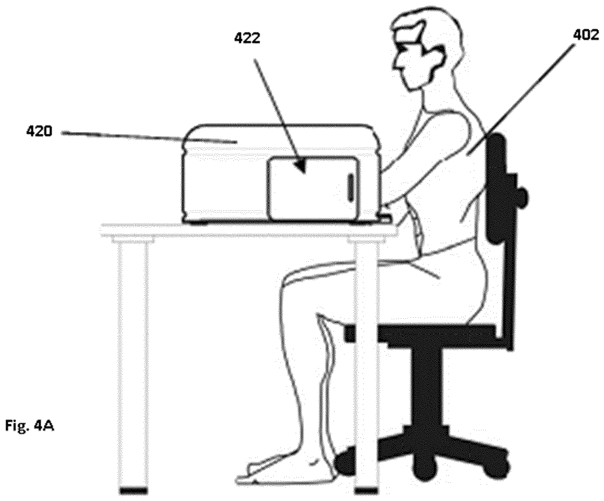

Two wheals W1 and W2 may be identified in this three-dimensional representation. Fig. 4A illustrates a usage scenario of an alternative device for measuring wheals. The device 400 may comprise a casing or housing 420 having an access door 422 on one side. The access door 422 is depicted closed in Fig. 4A. The purpose of the case may be to protect the eyes of the patient or of any other person in the vicinity, such as the medical personnel, from having direct contact with the light beam, as the line beam may be a laser beam or a led beam.

Although no harm to the eyes or any other organ is anticipated by the use of examples of the devices described herein, a casing or housing could be provided for psychological purposes as the patients may not be comfortable with facing a laser beam and this may cause agitation and unsteadiness that may compromise the accuracy of the measurements.

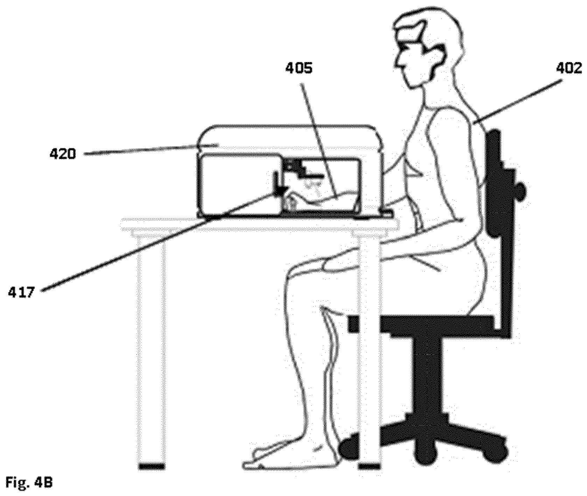

Fig. 4B illustrates the device 400 with the side door 422 open. The forearm 405 of the person 402 is in the resting place of the device and is ready to be treated by the head unit 417.

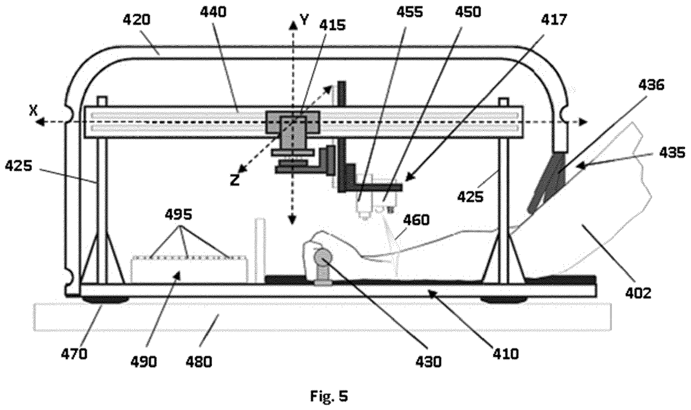

Fig. 5 is a cross-section of the casing 420 of the device 400. The device may comprise a resting base 410 having a pair of lower stops 470 of elastic material, e.g. rubber, which may provide dynamic stability to the device while ensuring proper fixation on a support surface 480 to prevent any undesirable movement occurring during the allergy test or the wheal measuring process.

The device illustrated in Fig. 5 may comprise an inlet area 435 where the arm of the person 402 may be introduced. The inlet area may be closed with a flexible curtain 436 that may allow the entrance of the arm of the patient but prevent the emission of e.g. a laser beam radiations outwards.

A guide 440 may be fixed upon two columns 425. The guide may be substantially along a first direction X, the first direction substantially coinciding with the longitudinal axis of the forearm of the patient. A movable unit 415 may be guidable along the first direction on the guide 440. The movable unit 415 may comprise a first portion movable along the longitudinal axis X of guide 440 and a second portion, a head 417, which may be movable along a second transversal axis Y and along a vertical axis Z. The head 417 may be movable relative to the first portion of the movable unit 415.

The head unit 417 may comprise a light beam generator, such as a laser line generator, for emitting a laser beam line 460 towards the forearm of the patient and a reflection receiver for receiving the reflection of the beam line.

The head unit 417 may further comprise an allergen dispensing module 455. When the forearm is introduced into the device, allergen dispensing module may provide an allergen on a point of the forearm. The device may further comprise an incision tool on the head 417 to perform an incision at the point where the allergen was deposited.

In an alternative, non-illustrated example, a first head unit may comprise e.g. a light beam generator and a suitable sensor, and a separate second head unit may comprise an allergen dispensing module and optionally an incision tool.

Providing the allergen on the forearm of the patient may be performed, as in the conventional method, by placing a drop of allergen to the skin of the patient and performing a micro-incision to the skin at the point where the drop of the allergen has been deposited. Then, after waiting for a few minutes, a reaction papule or wheal may appear as a positive result on the test patient's skin.

The forearm may then be scanned, to generate a three-dimensional representation of the area of the forearm and a set of filters may be applied on the three dimensional representation of the forearm to generate a three dimensional representation of the papules.

The disclosed system may comprise a computer system having a computer program to perform the scanning, the digitization of the forearm and the subsequent filtering and measurement or it may be connected to a computer system having a computer program to perform the aforementioned analysis. The device 400 may further comprise an allergen storage module 490 having a plurality of storage spaces for holding a set of single dose capsules 495 that may be fitted to allergen dispensing module 455.

In an alternative implementation, the device 400 may comprise a storage module 490 that may comprise multiple reservoirs, each filled with a particular allergen. The head unit 417 may move towards the storage module 490, so that the allergy dispensing module 455 may be recharged with allergens, thus avoiding the need to employ conduits.

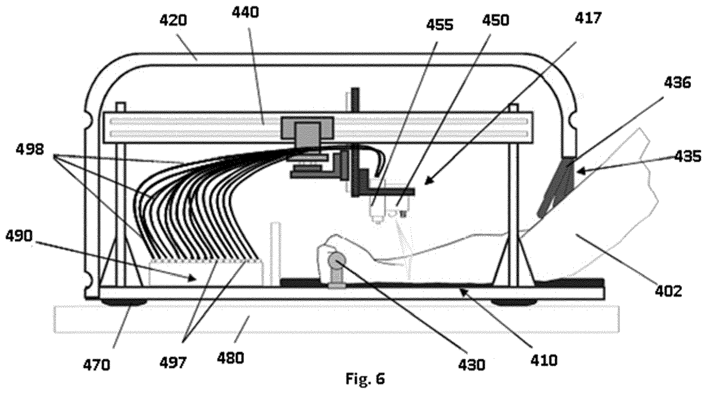

In another example, as it is shown in Fig. 6, the storage module 490 may comprise multiple reservoirs 497, each filled with a given allergen, and each reservoir 497 may be connected by a respective conduit 498 with the allergen dispensing module 455. The allergen dispensing module may be controllable by a computer. When a given allergen test is selected by a medical practitioner, the computer may identify the reservoir with the selected allergen and couple the respective conduit to the allergen dispensing module 455 by, e.g. controlling a respective valve (not shown). If a series of tests is selected then the computer may perform a series of allergen selection and conduit coupling and, at the same time, displace the movable unit so that each allergen is provided to a different point on the forearm of the patient.

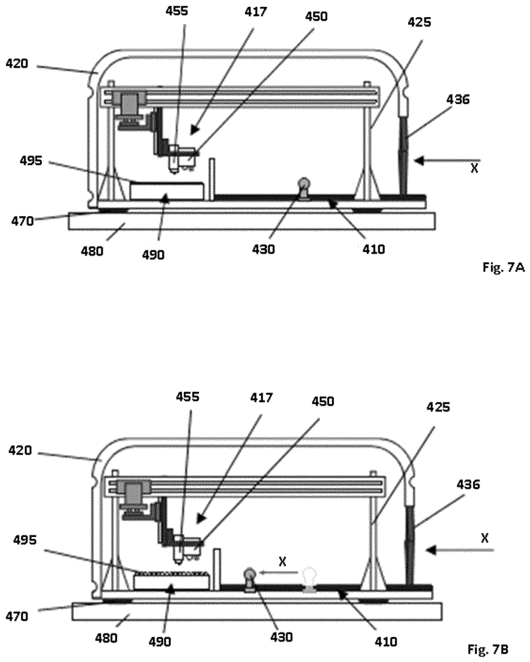

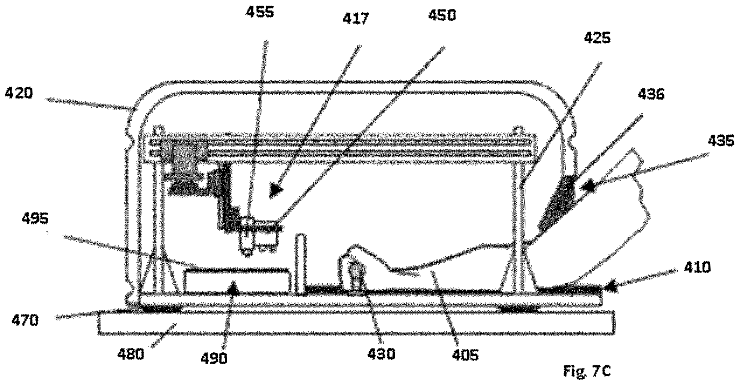

Fig. 7A to 7E illustrate an operating sequence of an allergy measuring device according to an example. In Fig. 7A the device is shown having the head unit 417 in resting position. In Fig. 7B, the handle 430 is moved to operating position. In Fig. 7C, the arm of a patient is introduced in the device and the patient grabs the handle 430.

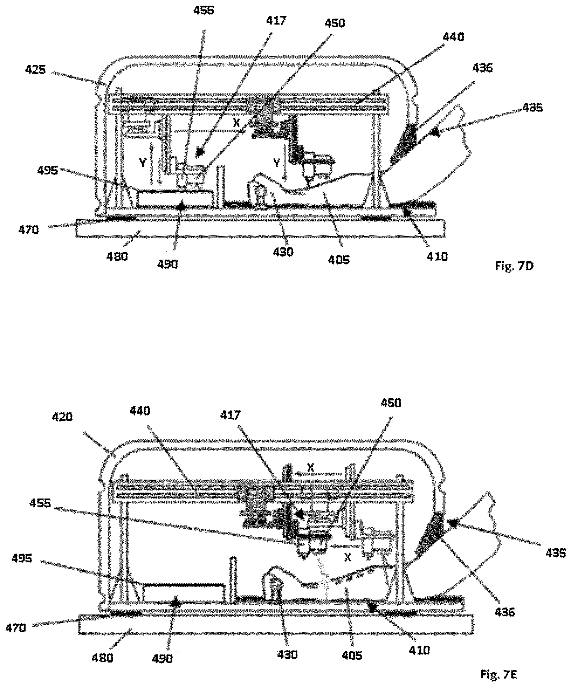

Fig. 7D illustrates the provision of the allergen on the skin of the person by the device. It may comprise both the provision of the allergen by the allergen dispensing module 455 and the performing of the incision by an incision tool. The movable unit moves towards the storage area 490 and the allergen dispensing module selects a single dose capsule 495 hosting the proper allergen. According to another implementation it may be refilled with the proper allergen.

Then the movable unit may move in the direction X towards the forearm of the patient. The head unit 417 may then be lowered, in a Y direction, towards the forearm to provide the allergen and/or perform the incision. Fig. 7E shows the final operating step of the device while the forearm of the person is in the device. A number of wheals are illustrated in the figure. The movable unit moves along the guide 440 and the two-dimensional sensor scans the forearm by generating light beams, e.g. laser line beams, along the area of the forearm where the wheals may be present.

The scanning may generate a plurality of two-dimensional sections of the area of the forearm which may be used by a computing system, internal or external to the device, to generate a three-dimensional representation of the forearm.

To facilitate detection of the wheals, it is also possible to mark with a pen or marker an outline of the wheal, as in the conventional method, and then scan in order to detect the mark, so that the computer system recognizes the shape and size of the outline of the mark, integrating the result into the application. This may allow the computer application to associate the digitized measurement of the allergic wheal with the allergen, so that the computer program may calculate automatically e.g. a perimeter, an area or other dimensions of the allergic wheal, saving this information in the computer system, where the information may remain stored in the medical history of the patient, and thereby being accessible from any point connected to the system.

The disclosed methods and devices are not limited to the detection of allergies, and may have more extensive applications to other conditions susceptible to be analyzed for their shape and size, such as moles, warts, etc.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

Further, although the embodiments of the invention described with reference to the drawings comprise computer apparatus and processes performed in computer apparatus, the invention also extends to computer programs, particularly computer programs on or in a carrier, adapted for putting examples of the disclosure into practice. The program may be in the form of source code, object code, a code intermediate source and object code such as in partially compiled form, or in any other form suitable for use in the implementation of the processes according to the invention. The carrier may be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means.

When the program is embodied in a signal that may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

## Claims

1. A method of measuring one or more dimensions of one or more wheals (W1, W2) on a three-dimensional representation (300) of an area of a skin of a forearm of a person executed by a processor of a computer device, comprising:
applying (110) a filtering stage to the three-dimensional representation of the area of the skin of the person to generate a first and a second intermediate representations of the area, wherein said filtering stage is configured to filter out (115) any irregularities of the three-dimensional representation below a first predetermined size to generate the first intermediate representation; and wherein said filtering stage is configured to filter out (120) any irregularities of the three-dimensional representation below a second predetermined size to generate the second intermediate representation;
subtracting (130) the second intermediate representation of the area from the first intermediate representation of the area to generate a three-dimensional representation of said one or more wheals;
determining (140) one or more dimensions of said three-dimensional representation of said one or more wheals.

2. The method according to claim 1, wherein said applying a filtering stage comprises
applying at least a first filtering stage to the three-dimensional representation of the area of the skin of the person to generate the first intermediate representation of the area; and
applying at least a second filtering stage to the first intermediate representation of the area of the skin to generate the second intermediate representation of the area.

3. The method according to claim 2, wherein the first predetermined size is substantially smaller than a minimum size wheal.

4. The method according to any of claims 1 to 3, wherein the first predetermined size is a predetermined length of approximately 3 mm and/or a surface of approximately 7mm².

5. The method according to any of claims 3 - 4, wherein the second predetermined size is larger than a maximum size wheal.

6. The method according to any of claims 1 - 5, further comprising scanning the area of the skin with a light beam to generate said three-dimensional representation of the area of the skin.

7. The method according to claim 6, wherein said scanning comprises:
displacing a light beam generator (215A) in a direction along the axis of the forearm (205; 405);
emitting a light beam from said light beam generator;
receiving a reflection of said light beam;
applying a three-dimensional triangulation algorithm to generate said three-dimensional representation of the area of the skin.

8. The method according to claim 7, wherein the light beam (260) projected onto the skin corresponds to a line projected onto the skin, and wherein a two-dimensional representation of a plurality of sections of the forearm are obtained and wherein the three-dimensional representation is based on the plurality of two-dimensional representations of sections of the forearm.

9. A device (200; 400) for measuring wheals comprising:
a resting base (210; 410) to receive a forearm (205; 405) of a person (402) in a first direction;
a light beam generator (215A) for emitting a light beam, the light beam generator being movable along the first direction, and a sensor (215B) that is operatively coupled to the light beam generator such as to move with the light beam generator and to receive a reflection from the light beam emitted onto the forearm,
wherein the device is configured to generate a three-dimensional representation (300) of at least a portion of said forearm (405), further comprising a filtering module configured to receive the three-dimensional representation of the portion of the forearm (405) and generate a three-dimensional representation (300) of the one or more wheals (W1, W2), wherein
the filtering module is configured to apply a filtering stage to the three-dimensional representation of the portion of the forearm to generate a first intermediate representation of the area, the first intermediate representation having filtered out any features of the skin having a size substantially smaller than that of the one or more wheals; and
generate a second intermediate representation of the area, the second intermediate representation having filtered out any features of the skin having a size up to the substantially the size of the one or more wheals;
a subtraction module, configured to subtract the second intermediate representation of the area from the first intermediate representation of the area to generate a three-dimensional representation of the one or more wheals.

10. The device according to claim 9, further comprising at least one guide (240; 440), substantially parallel to the first direction, wherein the light beam generator is movable along said at least one guide.

11. The device according to claim 9 or 10, wherein the light beam generator comprises a light source, wherein the light source is a laser or a LED.

12. The device according to any of claims 9-11, further comprising an incision tool (450), configured to automatically perform an incision at a selected point of the forearm and/or an allergen dispensing module (455) configured to automatically provide an allergen on the selected point of the forearm.

13. The device according to claim 9, wherein the filtering module is configured to apply smoothening filters.

14. A computing device comprising a non-transitory memory and a processor, wherein the memory stores computer program instructions executable by the processor, said instructions comprising functionality to execute a method of measuring one or more wheals on a three-dimensional representation of an area of the skin of a forearm of a person according to any of claims 1 to 8.

15. A computer program product comprising program instructions, the program instructions when executed on a computer system causing a computer system to perform the method of any of claims 1 - 8.

## Patentansprüche

1. Ein Verfahren zur Messung von einer oder mehreren Abmessungen von einer oder mehreren Urtikä (W1, W2) auf einer dreidimensionalen Darstellung (300) von einem Hautbereich eines Unterarms von einem Menschen, die durch einen Prozessor eines Rechners durchgeführt worden ist, umfassend:
das Anwenden (110) von einem Filterschritt auf die dreidimensionale Darstellung des Hautbereiches des Menschen, um eine erste und eine zweite mittlere Darstellungen des Bereiches zu erzeugen, wobei der Filterschritt konfiguriert ist, um jede Unregelmäßigkeit der dreidimensionalen Darstellung unterhalb von einer ersten vorherbestimmten Größe auszufiltern (115), um die erste mittlere Darstellungen zu erzeugen; und wobei der Filterschritt konfiguriert ist, um jede Unregelmäßigkeit der dreidimensionalen Darstellung unterhalb von einer zweiten vorherbestimmten Größe auszufiltern (120), um die zweite mittlere Darstellung zu erzeugen;
das Subtrahieren (130) der zweiten mittleren Darstellung des Bereiches von der ersten mittleren Darstellung des Bereiches, um eine dreidimensionale Darstellung von der einen oder den mehreren Urtikä zu erzeugen;
das Feststellen (140) von einer oder von mehreren Abmessungen der dreidimensionalen Darstellung von der einen oder den mehreren Urtikä.

2. Das Verfahren nach Anspruch 1, wobei der Schritt, in dem ein Filterverfahren angewendet wird folgendes umfasst:
das Anwenden von mindestens einem ersten Filterschritt auf die dreidimensionale Darstellung des Hautbereiches des Menschen, um die erste mittlere Darstellung des Bereiches zu erzeugen; und
das Anwenden von mindestens einem zweiten Filterschritt auf die erste mittlere Darstellung des Hautbereiches, um die zweite mittlere Darstellung des Bereiches zu erzeugen.

3. Das Verfahren nach Anspruch 2, wobei die erste vorherbestimmte Größe im Wesentlichen kleiner als eine Mindesturtikagröße ist.

4. Das Verfahren nach einem der Ansprüche 1 bis 3, wobei die erste vorherbestimmte Größe eine vorherbestimmte Länge von ca. 3 mm und/oder eine Fläche von ca. 7 mm² ist.

5. Das Verfahren nach einem der Ansprüche 3 - 4, wobei die zweite vorherbestimmte Größe länger als eine Höchsturtikagröße ist.

6. Das Verfahren nach einem der Ansprüche 1 - 5, weiterhin umfassend das Abtasten des Hautbereiches mit einem Lichtstrahl, um die dreidimensionale Darstellung des Hautbereiches zu erzeugen.

7. Das Verfahren nach Anspruch 6, wobei das Abtasten folgendes umfasst:
das Verschieben von einem Lichtstrahlengenerator (215A) in einer Richtung entlang der Achse des Unterarms (205; 405);
das Absenden von einem Lichtstrahl aus dem Lichtstrahlengenerator;
das Empfangen von einer Reflexion des Lichtstrahles;
das Anwenden von einem dreidimensionalen Triangulationsalgorithmus, um die dreidimensionale Darstellung des Hautbereiches zu erzeugen.

8. Das Verfahren nach Anspruch 7, wobei der auf die Haut projizierte Lichtstrahl (260) einer auf die Haut projizierten Linie entspricht, und wobei eine zweidimensionale Darstellung von einer Vielzahl von Abschnitten des Unterarms erhalten werden und wobei die dreidimensionale Darstellung auf der Vielzahl von zweidimensionalen Darstellungen von Abschnitten des Unterarms beruht.

9. Eine Vorrichtung (200; 400) zur Messung von Urtikä umfassend:
eine Auflagefläche (210; 410), um einen Unterarm (205; 405) von einem Menschen (402) in einer ersten Richtung zu empfangen;
einen Lichtstrahlengenerator (215A) zur Emission von einem Lichtstrahl, wobei der Lichtstrahlengenerator entlang der ersten Richtung bewogen werden kann, und einen Sensor (215B), der an dem Lichtstrahlengenerator betriebsfähig gekoppelt ist, um mit dem Lichtstrahlengenerator bewogen zu werden und um eine Reflexion von dem auf den Unterarm emittierten Lichtstrahl zu empfangen,
wobei die Vorrichtung konfiguriert ist, um eine dreidimensionale Darstellung (300) von mindestens einem Teil des Unterarms (405) zu erzeugen, weiterhin umfassend ein Filtermodul, das konfiguriert ist, um die dreidimensionale Darstellung des Teils des Unterarms (405) zu empfangen und eine dreidimensionale Darstellung (300) von der einer oder den mehreren Urtikä (W1, W2) zu erzeugen, wobei
das Filtermodul konfiguriert ist, um einen Filterschritt auf die dreidimensionale Darstellung des Teils des Unterarms anzuwenden, um eine erste mittlere Darstellung des Bereiches zu erzeugen, wobei die erste mittlere Darstellung jede Eigenschaft der Haut ausgefiltert hat, deren Größe im Wesentlichen kleiner als die Größe von der einen oder den mehreren Urtikä ist; und
das Erzeugen von einer zweiten mittleren Darstellung des Bereiches, wobei die zweite mittlere Darstellung jede Eigenschaft der Haut ausgefiltert hat, deren Größe im Wesentlichen bis zu der Größe von der einen oder den mehreren Urtikä ist;
ein Subtraktionsmodul, das konfiguriert ist, um die zweite mittlere Darstellung des Bereiches von der ersten mittleren Darstellung des Bereiches zu subtrahieren, um eine dreidimensionale Darstellung von der einen oder den mehreren Urtikä zu erzeugen.

10. Die Vorrichtung nach Anspruch 9, weiterhin umfassend mindestens eine Führung (240; 440), die im Wesentlichen parallel zu der ersten Richtung ist, wobei der Lichtstrahlengenerator entlang der mindestens einen Führung bewogen werden kann.

11. Die Vorrichtung nach Anspruch 9 oder 10, wobei der Laserstrahlengenerator eine Lichtquelle umfasst, wobei die Lichtquelle ein Laser oder ein LED ist.

12. Die Vorrichtung nach einem der Ansprüche 9 - 11, weiterhin umfassend ein Schneideinstrument (450), das konfiguriert ist, um einen Einschnitt in einen ausgewählten Punkt des Unterarms durchzuführen und/oder ein Allergenabgabemodul (455), das konfiguriert ist, um ein Allergen auf den ausgewählten Punkt des Unterarms abzugeben.

13. Die Vorrichtung nach Anspruch 9, wobei das Filtermodul konfiguriert ist, um Glättungsfilter anzuwenden.

14. Ein Rechengerät umfassend einen nicht-transitorischen Speicher und einen Prozessor, wobei der Speicher Computerprogrammanweisungen speichert, die durch den Prozessor ausgeführt werden können, wobei die Anweisungen Funktionalität zur Ausführung von einem Verfahren zur Messung von einer oder mehreren Urtikä auf einer dreidimensionalen Darstellung von einem Hautbereich von einem Unterarm eines Menschen nach einem der Ansprüche 1 bis 8 umfassen.

15. Ein Computerprogrammprodukt umfassend Programmanweisungen, wobei die Programmanweisungen die Durchführung des Verfahrens von einem der Ansprüche 1 - 8 durch ein Computersystem herbeiführen, wenn sie auf einem Computersystem ausgeführt werden.

## Revendications

1. Un procédé de mesure d'une ou de plusieurs dimensions d'une ou de plusieurs papules (W1, W2) sur une représentation tridimensionnelle (300) d'une région de la peau d'un avant-bras d'une personne exécutée moyennant un processeur d'un dispositif informatique, comprenant :
appliquer (110) une étape de filtrage à la représentation tridimensionnelle de la région de la peau de la personne afin de générer une représentation intermédiaire première et seconde de la région, dans lequel ladite étape de filtrage est configurée pour filtrer (115) toute irrégularité de la représentation tridimensionnelle au-dessous d'une première taille prédéterminée afin de générer la première représentation intermédiaire ; et dans lequel ladite étape de filtrage est configurée pour filtrer (120) toute irrégularité de la représentation tridimensionnelle au-dessous d'une seconde taille prédéterminée afin de générer la seconde représentation intermédiaire ;
soustraire (130) la seconde représentation intermédiaire de la région de la première représentation intermédiaire de la région afin de générer une représentation tridimensionnelle de ladite une ou desdites plusieurs papules ;
déterminer (140) une ou plusieurs dimensions de ladite représentation tridimensionnelle de ladite une ou desdites plusieurs papules.

2. Le procédé selon la revendication 1, dans lequel ladite application d'une étape de filtrage comprend
appliquer au moins une première étape de filtrage à la représentation tridimensionnelle de la région de la peau de la personne afin de générer la première représentation intermédiaire de la région ; et
appliquer au moins une seconde étape de filtrage à la première représentation intermédiaire de la région de la peau afin de générer la seconde représentation intermédiaire de la région.

3. Le procédé selon la revendication 2, dans lequel la première taille prédéterminée est essentiellement plus petite qu'une papule de taille minimale.

4. Le procédé selon l'une quelconque des revendications 1 à 3, dans lequel la première taille prédéterminée est une longueur prédéterminée d'environ 3 mm et/ou une surface d'environ 7 mm².

5. Le procédé selon l'une quelconque des revendications 3 - 4, dans lequel la seconde taille prédéterminée est plus grande qu'une papule de taille maximale.

6. Le procédé selon l'une quelconque des revendications 1 - 5, comprenant en outre balayer la région de la peau avec un faisceau de lumière afin de générer ladite représentation tridimensionnelle de la région de la peau.

7. Le procédé selon la revendication 6, dans lequel ledit balayage comprend :
déplacer un générateur de faisceaux de lumière (215A) dans une direction le long de l'axe de l'ante-bras (205 ; 405) ;
émettre un faisceau de lumière dudit générateur de faisceaux de lumière ;
recevoir une réflexion dudit faisceau de lumière ;
appliquer un algorithme de triangulation tridimensionnelle pour générer ladite représentation tridimensionnelle de la région de la peau.

8. Le procédé selon la revendication 7, dans lequel le faisceau de lumière (260) projeté sur la peau correspond à une ligne projetée sur la peau, et dans lequel une représentation bidimensionnelle d'une pluralité de sections de l'ante-bras sont obtenues et dans lequel la représentation tridimensionnelle est réalisée sur la base de la pluralité de représentations bidimensionnelles de sections de l'ante-bras.

9. Un dispositif (200 ; 400) pour mesurer des papules comprenant :
une base d'appui (210 ; 410) pour recevoir un ante-bras (205 ; 405) d'une personne (402) dans une première direction ;
un générateur de faisceaux de lumière (215A) pour émettre un faisceau de lumière, le générateur de faisceaux de lumière étant déplaçable le long de la première direction, et un capteur (215B) qui est couplé de façon opérationnelle avec le générateur de faisceaux de lumière de façon qu'il se déplace avec le générateur de faisceaux de lumière et de façon à recevoir une réflexion du faisceau de lumière émis sur l'ante-bras,
dans lequel le dispositif est configuré pour générer une représentation tridimensionnelle (300) d'au moins une partie dudit ante-bras (405), comprenant en outre un module de filtrage configuré pour recevoir la représentation tridimensionnelle de la partie de l'ante-bras (405) et générer une représentation tridimensionnelle (300) de l'une ou des plusieurs papules (W1, W2), dans lequel
le module de filtrage est configuré pour appliquer une étape de filtrage à la représentation tridimensionnelle de la partie de l'ante-bras afin de générer une première représentation intermédiaire de la région, la première représentation intermédiaire ayant filtré toute caractéristique de la peau ayant une taille essentiellement plus petite que celle de l'une ou des plusieurs papules ; et
générer une seconde représentation intermédiaire de la région, la seconde représentation intermédiaire ayant filtré toute caractéristique de la peau ayant une taille jusqu'à essentiellement la taille de l'une ou des plusieurs papules ;
un module de soustraction, configuré pour soustraire la seconde représentation intermédiaire de la région de la première représentation intermédiaire de la région afin de générer une représentation tridimensionnelle de l'une ou des plusieurs papules.

10. Le dispositif selon la revendication 9, comprenant en outre au moins une guide (240 ; 440), essentiellement parallèle à la première direction, dans lequel le générateur de faisceaux de lumière est déplaçable le long de ladite au moins une guide.

11. Le dispositif selon la revendication 9 ou 10, dans lequel le générateur de faisceaux de lumière comprend une source de lumière, dans lequel la source de lumière est un laser ou une LED.

12. Le dispositif selon l'une quelconque des revendications 9 - 11, comprenant en outre un outil d'incision (450), configuré pour effectuer de manière automatique une incision dans un point choisi de l'ante-bras et/ou un module d'administration d'allergènes (455) configuré pour fournir de manière automatique un allergène sur le point choisi de l'antrebras.

13. Le dispositif selon la revendication 9, dans lequel le module de filtrage est configuré pour appliquer des filtres de lissage.

14. Un dispositif informatique comprenant une mémoire non transitoire et un processeur, dans lequel la mémoire stocke des instructions de programme informatique exécutables par le processeur, lesdites instructions comprenant de la fonctionnalité pour exécuter un procédé de mesure d'une ou de plusieurs papules sur une représentation tridimensionnelle d'une région de la peau d'un ante-bras d'une personne selon l'une quelconque des revendications 1 à 8.

15. Un produit de programme informatique comprenant des instructions de programme, les instructions de programme, lors de leur exécution sur un système informatique, aboutissant à ce qu'un système informatique effectue le procédé de l'une quelconque des revendications 1 - 8.
